# EUROPEAN PATENT APPLICATION

(11) **EP 1 384 716 A1**
(43) Date of publication of application: **28.01.2004**
(21) Application number: 02078060.7
(22) Date of filing: 25.07.2002
(51) Int. Cl.: C07D 241/06, C07D 241/12, A23L 1/234, A23L 1/236

(54) **Preparation of ethenylpyrazines**

(71) Applicant: Nestec S.A., 1800 Vevey (CH)
(72) Inventor: Kurniadi, Toshinari, 1510 Moudon (CH)
(74) Representative: Archambault, Jean

(57) **Abstract**

The present invention relates to a new process for the generation of ethenylpyrazine derivative compounds consisting in reacting at ambient conditions a diketone of the formula CH2-CH-CO-CO-CH3 with 1,2-diaminopropane. Such ethenylpyrazine derivative compounds exhibit roasted and earthy aroma profiles and may be used in the food and beverage industry, especially chocolate, confectionery and coffee.

## Description

The present invention relates to the preparation of ethenylpyrazines compounds and derivatives thereof as well as compositions containing the same.

Pyrazines are heterocyclic, nitrogen containing compounds which contribute significantly to the flavour of many foods.
Pyrazine compositions and derivatives thereof are known to have utility in varieties of applications is various industries as broad-ranging as the pharmaceutical, tobacco, dyestuff and of course food industries.
Pyrazines derivatives have been known to impart a regular coffee flavor generally described as green by experts as evidenced in the British patent 1 156 475.
US 3622346 discloses the unique nutty, roasted flavors of the methoxypyrazines having potential applications as additives to a variety of baked or toasted foodstuffs.
A row of patents involving the use of pyrazines as flavour enhancers in foods indicate their potential as flavouring agents. Such patents include the addition of pyrazines for popcorn-like, nut-like, coffee-like, pineapple-like and chocolate-like flavours (US 3402051, US 3619210, US 3803331 and GB 1248380).
A plurality of reactants compounds, compositions, reactions mecanisms and conditions have been proposed and suggested in the art for the manufacture of pyrazines and derivatives.
US 3067199 describes the reaction of alkanolamine with nickel or cobalt hydrogenation/dehydrogenation catalysts at elevated temperature under subatmospheric pressure.

In US 3676442, acetylene compounds are reacted with an ammoniacal derivative under acid conditions at elevated pressure.
Japanese Kokai application 52-97983 discloses a process where *hydroxyketone* compound and an ammonium salt of an acid are heated with a heat-stable solvent under neutral or acidic conditions.
As indicated by Rizzi (J. Agric. Food Chem. Vol. 36 N°2, 1988, pp 349-352) who proceed to study the nature of pyrazine formation from *hydroxyketone* compounds and ammonium salts by reaction under reflux conditions, although alkyl pyrazines have been widely investigated as trace components in foods, their exact origin remains unclear. Particularly, Rizzi noted that alkyl pyrazines have been found in fermented products and it is also believe that they are formed during baking, roasting, cooking of various foodstuffs and have consequently great importance as flavor components of such foodstuffs. In particular, it is believed that these compounds impart roast flavor and aromatic characteristic to foodstuffs. Thus efforts are being made to attempt to prepare such compositions in a food acceptable manner.

As noted from all the above mentionned prior art, the procedures to manufacture and isolate pyrazines compounds are generally complex, cumbersome, involve catalysts, solvents, high temperature and/or high pressure conditions, heavy equipement for performing the reaction ...

It thus appears a need for a simple, cheap, direct and food-grade process for the generation of pyrazines compounds.

Accordingly, the present invention provides a process for the preparation of ethenylpyrazine derivatives compounds comprising the steps of reacting at ambient conditions a diketone with 1,2-diaminopropane, in order to obtain ethenyl pyrazine derivative compounds.

The preferred diketone may be selected in the group comprising a diketone of the formula CH2-CH-CO-CO-CH3, 4-penten-2,3-dione.

The expression "ambient conditions" refers to ambient temperature and pression conditions, namelly a temperature ranging between about 15°C and 25°C, and a pression of about the atmospheric pressure, about 950 to 1050 hPa, for exemple.

The concentrations of the starting products in the medium, e.g. diketone and 1,2-diaminopropane (DAP) may be in the range of 10 mM to 1000 mM for both reactants.

Regarding the respective quantities of the starting products diketone and 1,2-diaminopropane, they can be such that the molar ratio diketone:1,2-diaminopropane may vary from about 1:2 to 2:1. Optimal ratio diketone:1,2-diaminopropane will depend on the relative aboundance of the desired isomer.

The reaction of the diketone with 1,2-diaminopropane (DAP) in order to produce ethenlpyrazine derivatives may be achieved in an aqueous based medium but also in an other non aqueous medium like ether, chloroform, ethanol or methanol for exemple.

The reaction of diketone with DAP may be done at room temperature, such as from 18 to 25°C for exemple, but it may be performed at higher temperatures in order to accelerate the reaction. Indeed it is the first time that generation of ethenylpyrazines compounds is obtained by a chemical reaction at such low temperatures. The reaction may be carried out at such temperature during a time sufficient to generate the ethenylpyrazines derivatives, for instance during from about 1 hour to about 20 hours depending on the temperature used. The aqueous phase containing the ethenylpyrazine derivatives may be extracted with diethyl ether for example for characterization by means of gas chromatography. The extraction procedure may be repeated twice or three times.

Using a diketone of the formula CH2-CH-CO-CO-CH3, GC-MS analysis revealed that in all solvents two molecules with a molecular mass of 136 were obtained. The mass fragmentation pattern indicated that the compounds are 2-ethenyl-3,5-dimethyl-5,6-dihydropyrazine and are 2-ethenyl-3,6-dimethyl-5,6-dihydropyrazine.

New roasted or earthy aroma properties are for example interesting for beverage industry as well as chocolate and confectionery one. Accordingly it is also an object of the present invention to use of the ethenylpyrazines derivatives according to the present invention in food products, especially beverages for purpose of taste and flavour improvement.
It is also an object of the present invention to provide flavouring composition containing ethenylpyrazine derivatives compounds obtainable by a process as described above.

### Materials

All chemicals were from Sigma Aldrich Chemical Co.

### Methods

### Gas Chromatography (GC) and Gas Chromatography / Olfactometry (GC-O)

GC-analyses were performed on a Agilent 6890 Series GC equipped with a Splitless injector, a flame ionization detector (FID) and sniffing port. Separation of volatiles was either achieved on a DB-Wax capillary column or a DB-1 capillary column (30 m x 0.25 mm, film thickness 0.25 mm, J & W Scientific) using helium as carrier gas (1.5 mL min⁻¹) and nitrogen (45 kPa) as make up-gas for the FID. The following temperature programs were applied:
With DB-Wax:
   5 min isothermal at 40 °C, then raised to 220 °C at 5 °C/min and kept 5 min isothermal at 220 °C.
With DB-1:
   5 min isothermal at 40 °C, then raised to 240 °C at 4 °C/min and kept 15 min isothermal at 240 °C.

### Gas Chromatography/ Mass spectometry (GC/MS)

GC-MS analyses were performed on a Finnigan MAT-8430 mass spectrometer combined with an HP 5890 gas chromatograph using the same GC conditions as described above. The MS-EI spectra were generated at 70 eV and MS-CI at 150 eV with ammonia as reagent gas.

### Results and discussion

### Synthesis of 2-ethenyldimethyl-5,6-dihydropyrazines

300 mM 4-penten-2,3 dione was reacted with 330 mM 1,2 DAP and reaction was conducted for one hour at room temperature in water, ether, methanol and chloroform. GC-MS analysis revealed that in all the four used solvents, two molecules with the molecular mass of 135 were obtained. The mass fragmentation pattern indicated that the compounds were 2-ethenyl-3,5-dimethyl-5,6-dihydropyrazine and 2-ethenyl-3,6-dimethyl-5,6-dihydropyrazine.

## Claims

1. Process for the preparation of ethenylpyrazine derivatives compounds comprising the steps of reacting at ambient conditions a diketone with 1,2-diaminopropane, in order to obtain ethenyl pyrazine derivative compounds.

2. Process according to claim 1, **characterized in that** the diketone is selected in the group comprising a diketone of the formula CH2-CH-CO-CO-CH3.

3. Use of ethenylpyrazines derivatives compounds obtained according to claim 1 and 2 in food products, especially beverages for purpose of taste and flavour improvement.

4. Flavouring compositions containing ethenylpyrazines derivatives compounds obtainable according to claims 1 and 2.
